# EUROPEAN PATENT APPLICATION

(11) **EP 4 578 441 A1**
(43) Date of publication of application: **02.07.2025**
(21) Application number: 23220676.3
(22) Date of filing: 29.12.2023
(51) Int. Cl.: A61K 9/00, A61K 9/06, A61K 47/36

(54) **TREATMENT OF LOCALIZED PAIN**

(71) Applicant: PlantTec Medical GmbH, 21337 Lüneburg (DE)
(72) Inventor: LAAS, Joachim, 29549 Bad Bevensen (DE)
(74) Representative: Maikowski & Ninnemann Patentanwälte Partnerschaft mbB

(57) **Abstract**

This invention is in the field of anesthesiology and, in particular, the non-systemic or localized administration of pain medications to traumatized tissue for the treatment of localized pain. The invention provides a composition for use in methods for treating localized pain conditions after surgery. The invention further provides a process of preparing said composition. Furthermore, the invention provides the use of said composition in methods of treating, especially reducing, pain associated with a surgery.

## Description

### FIELD OF THE INVENTION

This invention is in the field of anesthesiology and, in particular, the non-systemic or localized administration of pain medications to traumatized tissue for the treatment of localized pain. The invention provides a composition for use in methods for treating localized pain conditions after surgery. The invention further provides a process of preparing said composition. Furthermore, the invention provides the use of said composition in methods of treating, especially reducing, pain associated with a surgery.

Pain can be defined as an unpleasant sensory and emotional experience associated with actual or potential tissue damage. It is a complex process influenced by both physiological and psychological factors. Pain is typically subjective, and many health care professionals are not trained to effectively assess or treat pain. The management of pain, particularly post- surgical pain, is most often based on the systemic administration of pain-relieving drugs.

Patients are entitled to appropriate pain therapy after surgery. Extensive studies have shown that despite today's improvements in pain management, many patients still suffer from moderate to severe post-operative pain (Jage, J. et al., Postoperative Schmerztherapie - eine interdisziplinäre Notwendigkeit. Dtsch Arztebl 2005; 102(6): A-361 / B-300 / C-282).

Relief from pain after surgery does not just mean a better subjective sense of well-being for patients. Pain can make patients ill. Alongside other perioperative factors, it is an intense stress factor and puts a strain on cardiopulmonary, endocrine, metabolic, gastrointestinal and immunological bodily functions as well as the psychological reaction to the operation. Pain-free patients have fewer complications and convalescence is accelerated.

Postoperative pain can develop into chronic pain. The extent to which effective perioperative pain therapy can prevent pain from becoming chronic is the subject of current studies.

Postoperative basic analgesia (i.e. systemic, i.e. oral/rectal/intravenous/subcutaneous administration of non-opioids/opioids) is difficult because, on the one hand, the patient's perception of pain and, on the other hand, the effect of pain medication varies greatly from individual to individual. Furthermore, all systemic analgesics have side effects such as nausea, cognitive impairment, dizziness or mild sedation. Opioids inhibit gastrointestinal activity, reduce vigilance and can lead to severe sedation and respiratory depression. Pain after surgery can often not be treated with basic analgesia alone.

The three major classes of pharmaceutical drugs used to treat post- surgical pain are the opiates, local anesthetics, and the non-steroidal anti- inflammatory drugs (NSAID). Two of these classes of drugs, the opiates and NSAIDs, are typically administered systemically while the local anesthetics (e. g. channel blockers) are administered non-systemically during surgery. The systemic administration of drugs to relieve pain after surgery is frequently inadequate. For example, systemic administration of opiates after surgery may cause nausea, the inhibition of bowel function, urinary retention, inhibition of pulmonary function, cardiovascular effects, and sedation. When NSAIDs are given systemically, there is a potential for gastrointestinal (GI) and renal side effects as well as inhibition of platelet function.

There are numerous references generally describing the administration of pain medications via drug delivery systems that may be utilized non-systemically.

The efficacy of various anesthetics for the treatment of post-surgical pain are also reported in many of these references. These systems typically consist of a polymeric matrix or liposome from which drug is released by diffusion and/or degradation of the matrix. The release pattern is usually principally determined by the matrix material, as well as by the percent of loading, method of manufacture, type of drug being administered and type or geometry of the device. Often the drug delivery system is biocompatible and absorbable or biodegradable, which permits it to be used intracorporeally at the surgical wound site or trauma site and then slowly absorbed or degraded by the patient's body. One advantage of using an absorbable or degradable drug delivery system at the surgical wound site or trauma site is that the site does not have to be re-opened to remove the drug delivery system after depletion of the drug.

As the clinical requirement for a drug delivery carrier of any given therapeutic agent for intra-corporeal application at a surgical wound site or a trauma site is much more demanding than for topical application of the same therapeutic agent, there is usually no predictive correlation between non-systemic topical application and non-systemic intra-corporeal application at a surgical wound site or a trauma site of a particular composition comprising a local anesthetic drug. In addition to drug-carrier compatibility, critical factors to consider for a drug delivery carrier for use in intra-corporeal application at a surgical wound site or a trauma site include, but is not limited to, tissue reaction, biocompatibility, bioabsorbability, biodegradability, and mechanical strength.

For example, one would not expect the mechanical properties of the composition disclosed in US 5,888,523 comprising an NSAID as a local anesthetic drug, to be suitable for intra-corporeal use at a surgical wound site or a trauma site, because the NSAID composition is based on a water dispersible cellulosic polymer that is not expected to remain in the fluid environment of the body for a long enough period of time to allow for release of the anesthetic drug, such as an NSAID.

EP2415487 A1 discloses an ultrasonic couplant composition comprising starch, a fast-acting local anaesthetic compound or a pharmaceutical acceptable salt thereof, a local anaesthetic compound with long lasting anaesthetic effect or a pharmaceutical acceptable salt thereof and an antiseptic compound or a pharmaceutical acceptable salt thereof. The suitability of this ultrasonic couplant composition for post-surgery administrations on wounds is not predictable.

WO08060365 A2 discloses a rather complex self-gelling tunable drug delivery system, which is comprised of a hydrophilic matrix and a hydrophobic matrix.

WO07100376 A2 is drawn to adhesive peel-forming formulations for dermal delivery of a drug. The formulation includes a drug, a solvent vehicle, and a peel-forming agent. The solvent vehicle can include a volatile solvent system having one or more volatile solvent, and a non-volatile solvent system having one or more non-volatile solvent, wherein the non-volatile solvent system has a solubility for the drug that is within a window of operable solubility for the drug such that the drug can be delivered at therapeutically effective rates over a sustained period of time. The formulation can have a viscosity suitable for application to a skin surface prior to evaporation of the volatile solvents system. When applied to the skin, the formulation can form a solidified peelable layer after at least a portion of the volatile solvent system is evaporated. These adhesive peel-forming formulations are, however, not bioabsorbable nor biodegradable.

WO15103450 A1 relates to an improved local anesthetic solution with diminished bitter taste includes an anesthetic agent, an anesthetic solution vehicle, and a bitterness suppressant. The bitterness suppressant includes one or more compounds selected from the group consisting of: a sugar selected from the group consisting of monosaccharide sugars, disaccharide sugars, polysaccharide sugars, and combinations of the any of the foregoing; sweet-tasting compounds; acids; amino acids; salts; miscellaneous suppressant substances; and combinations of any of the foregoing. The improved local anesthetic solution optionally includes one or more additional agents selected from the group consisting of: buffering agents; vasoconstrictors; preservative compounds; stabilizers; contrast media agents; and combinations of any of the foregoing. This local anesthetic solution is not suitable for treating post-surgery localized pain on wounds, where sustained release of a local anesthetic drug over a longer time period is required.

### Brief Summary of the Invention

As summarized hereinbefore, drug delivery systems comprising local anesthetics that may be utilized non-systemically, show certain disadvantages and there is need for improved local delivery systems for anesthetics.

To overcome the disadvantages of the prior art, the invention provides a composition for reducing pain at a surgical wound site or trauma site comprising starch and a pain medication, where the starch is a carboxymethylated starch or epichlorohydrin-modified starch.

In another embodiment, the carboxymethylated starch comprises citric acid or a salt of citrate salt.

In further embodiment, the anesthetic drug is ropivacaine.

Another embodiment of the invention is directed to the use of a composition comprising starch and a pain medication in a method for reducing pain at a surgical wound site or trauma site including, applying to the site an effective amount of a comprising starch and said pain medication, where the starch is carboxymethylated starch or epichlorohydrin-modified starch.

The present invention relates to a biocompatible and biodegradable composition useful for treating localized pain at a surgical wound site or trauma site. As used herein, "biocompatible" means that, once applied, the composition will not result in substantial tissue irritation or necrosis. The term "biodegradable" means that the composition will be degraded by the body over time by the action of enzymes, hydrolytic action or by contact with substances found in the surrounding tissue fluids or cellular action.

### Detailed description of the Invention

### Definitions

Unless defined otherwise, all technical and scientific terms used herein have the same meaning as is commonly understood by one skilled in the art to which this invention belongs.

As used herein and in the claims, the singular form "a," "an," and "the" includes plural references unless the context clearly dictates otherwise.

The terms "treatment," or "treating" or "ameliorating" as used in the specification and claims refer to an approach for achieving beneficial or desired results, including but not limited to a therapeutic benefit and/or a prophylactic benefit.

The term "therapeutic benefit" as used in the specification and the claims means eradication or amelioration of the underlying disorder being treated. A therapeutic benefit is achieved with the eradication or amelioration of one or more of the physiological symptoms associated with the underlying disorder such that an improvement is observed in the subject, notwithstanding that the subject may still be afflicted with the underlying disorder. For prophylactic benefit, the present compositions may be administered to a subject at risk of developing a particular affliction or disease, or to a subject reporting one or more of the physiological symptoms of a disease even though a diagnosis of the disease may not have been made.

The term "effective amount" refers to that amount of composition described herein that is sufficient to achieve the intended effect. The effective amount may vary depending upon the intended application or the subject and disease condition being treated, e.g., the weight and age of the subject, the severity of the disease condition, the manner of administration and the like, which can be determined readily by one skilled in the art. This term also applies to a dose that induces a particular response, e.g., reduction of pain. The specific dose may vary depending on the particular compounds that constitute the composition, the dosing regimen to be followed, timing of administration, and the physical delivery system in which the composition is carried.

As used herein, the term "non-systemic" refers to the application of a composition or drug to the site of a traumatized tissue, intracorporeally at a surgical wound site or a trauma site, or topically, i.e., internally and/or externally. The terms "surgical wound site" and "trauma site" are meant to include the site of tissue that has been injured in any way, and includes, for example, tissue sites that have undergone incision, drying, suturing, excision, abrasion, contusion, laceration, anastomosis, manipulation, prosthetic surgery, curettage, cardio-thoracic surgeries, ear-nose-throat surgeries, general surgeries, gynecological surgeries, neurosurgeries, surgeries in orthopedics and traumatology, urological surgeries, vascular surgeries, or plastic or reconstructive surgery. The treatment is intended to be "locally effective", that is the treatment is intended to affect only the tissue treated or adjacent or neighboring tissue.

Conditions, where the composition of the invention has been shown to be advantageous in pain treatment, are hysterectomy, endometriosis, ovarian cystectomy and caesarean section. Hysterectomy is the surgical removal of the uterus and cervix. Supracervical hysterectomy refers to removal of the uterus while the cervix is spared. These procedures may also involve removal of the ovaries (oophorectomy), fallopian tubes (salpingectomy), and other surrounding structures. These procedures are usually performed by a gynecologist. Removal of the uterus has surgical risks as well as long-term effects, so the surgery is normally recommended only when other treatment options are not available or have failed. It is the second most commonly performed gynecological surgical procedure, after cesarean section, in the United States.

Endometriosis is a disease of the female reproductive system in which cells similar to those in the endometrium, the layer of tissue that normally covers the inside of the uterus, grow outside the uterus. Lesions can be found on ovaries, fallopian tubes, tissue around the uterus and ovaries (peritoneum), intestines, bladder, and diaphragm; it may also occur in other parts of the body. Some symptoms include pelvic pain, heavy and painful periods, pain with bowel movements, painful urination, pain during sexual intercourse and infertility. Nearly half of those affected have chronic pelvic pain, while in 70% pain occurs during menstruation. Infertility occurs in up to half of affected individuals. About 25% of individuals have no symptoms and 85% of those seen with infertility in a tertiary center have no pain. Endometriosis can have both social and psychological effects.

The areas of endometriosis bleed each month (menstrual period), resulting in inflammation and scarring. The growths due to endometriosis are not cancer. Diagnosis is usually based on symptoms in combination with medical imaging; however, biopsy is the surest method of diagnosis. Other causes of similar symptoms include pelvic inflammatory disease, irritable bowel syndrome, interstitial cystitis, and fibromyalgia. Endometriosis is commonly misdiagnosed and females often report being incorrectly told their symptoms are trivial or normal. Females with endometriosis see an average of seven physicians before receiving a correct diagnosis, with an average delay of 6.7 years between the onset of symptoms and surgically obtained biopsies, the gold standard for diagnosing the condition. This average delay places endometriosis at the extreme end of diagnostic inefficiency.

Treatment consists of the ablation or excision of the endometriosis, electrocoagulation, lysis of adhesions, resection of endometriomas, and restoration of normal pelvic anatomy as much as is possible. When laparoscopic surgery is used, small instruments are inserted through the incisions to remove the endometriosis tissue and adhesions. Because the incisions are very small, there will only be small scars on the skin after the procedure, and most individuals recover from surgery quickly and have a reduced risk of adhesions. Many endometriosis specialists believe that excision is the ideal surgical method to treat endometriosis. As for deep endometriosis, a segmental resection or shaving of nodules is effective but is associated with an increased rate of complications, of which about 4.6% are major.

Historically, a hysterectomy (removal of the uterus) was thought to be a cure for endometriosis in individuals who do not wish to conceive. Removal of the uterus may be beneficial as part of the treatment, if the uterus itself is affected by adenomyosis. However, this should only be done in combination with removal of the endometriosis by excision. If endometriosis is not also removed at the time of hysterectomy, pain may persist. A study of hysterectomy patients found those with endometriosis were not using less pain medication 3 years after the procedure.

Presacral neurectomy may be performed where the nerves to the uterus are cut. However, this technique is not usually used due to the high incidence of associated complications including presacral hematoma and irreversible problems with urination and constipation.

An ovarian cystectomy is surgery to remove a cyst from an ovary. Usually, laparoscopic surgery, a minimally invasive surgery technique that only uses a few small incisions in the lower abdomen, is used to remove cysts from ovaries.

Cesarean section, C-section, or cesarean birth is the surgical delivery of a baby through a cut (incision) made in the mother's abdomen and uterus. Health care providers use it when they believe it is safer for the mother, the baby, or both. The incision made in the skin may be up-and-down (vertical). This incision extends from the belly button to the pubic hairline.

Alternatively, the incision is made across from side-to-side (horizontal). This incision extends across the pubic hairline. It is used most often, because it heals well and there is less bleeding. The type of incision used depends on the health of the mother and the fetus. The incision in the uterus may also be either vertical or horizontal.

Laparoscopic removal of ovarian cysts and caesarean section are the interventions with the highest reported pain scores (Cruz, J.J. et al., Acute postoperative pain in 23 procedures of gynaecological surgery analysed in a prospective open registry study on risk factors and consequences for the patient. Nature Scientific Reports (2021), 11:22148, https://doi.org/10.1038/s41598-021-01597-5).

As a result of the evaluations described in working examples hereinbelow, it has been shown that a composition comprising starch and an effective amount of a pain medication or pharmaceutically acceptable salt thereof to treat localized pain, where the starch is carboxymethylated or epichlorohydrin-modified starch starch, is surprisingly efficacious when administered non-systemically and intra-corporeally at the site of a surgical wound or trauma, for the reduction of localized pain at the site. In particular, the pain medication is selected from morphine, nonsteroidal anti-inflammatory drugs (NSAIDS), opioid analgesics (oxycodone, morphine, fentanyl, hydrocodone, naproxyphene, codeine, etc.), opioid/nonopioid combination analgesics (e.g. acetaminophen with codeine), acetaminophen, local anesthetics, alpha-2 agonists (clonidine, xylazine, medetomidine, dexmedetomidine), VR1 antagonists, and combinations thereof and the like.

Preferred pain medications for use in the composition of the invention are local anesthetics.

Preferred local anesthetics are selected from the group consisting of lidocaine, cocaine, procaine, and, ambucaine, amethocaine, amylocaine, articaine, betoxycaine, benzocaine, bupivacaine, levo-bupivacaine, butacaine, butanilicicaine, butoxycaine, butyl aminobenzoate, carticaine, cinchocaine, clibucaine, clormecaine, chloroprocaine, cyclomethycaine, dibucaine, dimethocaine, levo-etidocaine, etidocaine, dextro-etidocaine, beta-eucaine, etidocaine, fomocaine, hexylcaine, hydroxyprocaine, hydroxytetracaine, leucinocaine, levo-mepivacaine, mepivacaine, meprylcaine, metabutoxycaine, myrtecaine, octacaine, orthocaine, oxethazaine, oxybuprocaine, parethoxycaine, phenacaine, piperocaine, piridocaine, prilocaine, procaine, propanocaine, proparacaine, propipocaine, proxymetacaine, propoxycaine, pseudococaine, pyrrocaine, risocaine, ropivacaine, levo-ropivacaine, tetracaine, tolycaine, trimecaine, and vadocaine.

Most preferably, the local anesthetic used in the composition of the invention is ropivacaine.

It is understood that the present invention contemplates the use of not only the above-stated pain medications themselves, but their pro-drugs which metabolize to the compound and the analogues, biologically active salt forms and pharmaceutically acceptable salt forms thereof, as well as optical isomers which provide the same pharmaceutical results.

### The pharmaceutical composition

### A) Carboxymethylated or epichlorohydrin starch

A pharmaceutical composition useful for practicing the method of the invention comprises carboxymethylated starch or epichlorohydrin-modified starch, or a combination or mixture thereof. Surprisingly, it has been found that the administration of such a type of starch to a subject provides excellent results in the sustained release of the pain medication. This applies in particular for pain treatment following hysterectomy and ablation, excision or resection of the endometriosis, but also for any other type of surgeries with long lasting pain events.

The pharmaceutical composition can be advantageously present in the form of a gel; in addition, the pharmaceutical composition advantageously can include water and/or at least one salt, for example and in particular an alkali metal salt such as sodium chloride or potassium chloride.

In a preferred embodiment, the pharmaceutical composition comprises carboxymethylated starch, and optionally water and/or at least one alkali metal salt, e.g., sodium chloride or potassium chloride.

In another preferred embodiment, the pharmaceutical composition includes epichlorohydrin-modified starch, and optionally water and/or at least one alkali metal salt, e.g., sodium chloride or potassium chloride.

In a further preferred embodiment, the pharmaceutical composition comprises a combination or mixture of carboxymethylated starch and epichlorohydrin-modified starch, and optionally water and/or at least one alkali metal salt, which can be sodium chloride or potassium chloride.

In practice, it has proven beneficial for the carboxymethylated starch or the epichlorohydrin-modified starch to have a molecular weight of 500,000 daltons to 11,000,000 daltons, and/or for the starch particles, the carboxymethylated starch or the epichlorohydrin-modified starch particles to have a particle diameter in the range from 30 µm to 100 µm.

Accordingly, the invention provides a method of treatment utilizing pharmaceutical composition comprising carboxymethylated starch or epichlorohydrin-modified starch or a mixture or combination thereof, wherein said carboxymethylated starch or epichlorohydrin-modified starch has a molecular weight in the range from 500,000 daltons to 11,000,000 daltons, and/or wherein the starch particles of the carboxymethylated starch and/or the epichlorohydrin-modified starch particles have a particle diameter in the range of 30 microns to 100 microns.

Preferably, said carboxymethylated starch or epichlorohydrin-modified starch has a molecular weight in the range from 750,000 daltons to 10,000,000 daltons or 1,000,000 daltons to 9,000,000 daltons.

More preferably, said carboxymethylated starch or epichlorohydrin-modified starch has a molecular weight in the range from 2,000,000 daltons to 8,000,000 daltons or 3,000,000 daltons to 7,000,000 daltons.

Most preferably, said carboxymethylated starch or epichlorohydrin-modified starch has a molecular weight in the range from 4,000,000 daltons to 6,000,000 daltons.

Convincing results in the treatment of pain have been obtained with a pharmaceutical composition that comprises carboxymethylated starch and optionally water and/or at least one salt, selected from the group consisting of sodium chloride or potassium chloride, wherein said carboxymethylated starch has a molecular weight in the range from 500,000 daltons to 11,000,000 daltons, and/or wherein the starch particles of the carboxymethylated starch have a particle diameter of 30 microns to 100 microns.

The carboxymethylated starch of the pharmaceutical composition has the following, further preferred characteristics: It is a crosslinked, carboxymethylated starch containing approximately 20 wt % amylose and approximately 80 wt % amylopectin in a relatively constant ratio. The amylose is rather amorphous, and the amylopectin represents the crystalline portion of the starch. The modified starch is present as a salt-starch glycolate, and is generally the sodium salt of a carboxymethyl ether of the starch.

A starch particle of the carboxymethylated starch is made up of about 4.5×10¹⁰ to 2.3×10¹² amylose molecules and 5.6×10⁷ to 1.3×10¹⁰ amylopectin molecules. The degree of crosslinking of the carboxymethylated is typically in the range from 25to 45%.

The carboxymethylated starch may contain the at least one salt, which is selected from the group consisting of sodium chloride and potassium chloride, preferably sodium chloride, in an amount up to 10 weight %, preferably between 2 weight % and 5 weight %, calculated on the basis of the dry mass of the carboxymethylated starch.

After addition of water, or when the pharmaceutical composition is in gel form, the pH value of the solution or gel is in the range from 3.0 and 7.5, preferably from 5.0 to 7.5, most preferably from 6.5 to 7.5.

The carboxymethylated starch is present as a white or almost white fine, free-flowing powder. The powder is very hygroscopic, and is practically insoluble in methylene chloride. It forms a translucent suspension in water.

The powder is made up of irregularly shaped, oval or pear-shaped particles in a size range from 30 to 100 µm, or 10 to 35 µm with rounding.

The starch particles have typically a surface of approximately 0.2 m² /g. Occasionally occurring clusters of particles are made up of two to four particles. The particles have an eccentric hilus and clearly visible concentric grooves. The particles exhibit a distinct black cross on the hilus, between intersecting Nicol prisms. Small crystals are discernible at the surface of the particles. The particles exhibit significant swelling, up to approximately 30 times their own weight, upon contact with water or salt solutions.

Most preferred for the preparation of the pharmaceutical composition of the invention is the carboxymethylated starch that has the CAS Registry No. 9057-06-1.

The carboxymethylated starch comprised in the pharmaceutical composition can be produced using the following method:
The synthesis of the carboxymethylated starch generally takes place in three steps, in each case as a suspension, wherein Step 1 comprises crosslinking of the starch, Step 2 comprises carboxymethylation of the starch, and Step 3 is a neutralization step.

The crosslinking-step uses hydroxyl groups attached to starch (present in the basic glucose building blocks of the starch). The reaction of these hydroxyl groups with multifunctional reagents results in crosslinked starches. In a granule of starch, many chains are found in closer proximity, and such reactions not only take place between single chains, but it links side by side chains as well. These crosslinking agents form either ether or ester inter-molecular linkages between hydroxyl groups on starch molecules A small amount of multifunctional reagent is enough to interconnect starch molecules by crosslinking reactions. Methods for crosslinking of starches are generally known to the person skilled in the art. An overview of starch crosslinking is e.g. given in Shah, Nimish & Mewada, Rajubhai & Mehta, Tejal. (2016). Crosslinking of starch and its effect on viscosity behaviour. Reviews in Chemical Engineering. 32. 10.1515/revce-2015-0047, which is incorporated herein in entirety.

Since the reactions take place in suspension, the substitution is inhomogeneous. Outer areas of the starch are more greatly affected by the modifications than the core areas of the starch structures. The reactions are influenced by diffusion within the starch structure and accessibility to the starch structure. Amorphous areas are more easily accessible than crystalline areas, which are less strongly modified. From a chemical standpoint, both reactions are nonspecific, and, provided that diffusion inhibition or steric hindrance is present, have no preferred reaction pattern for the individual hydroxyl groups of a glucose unit. Theoretically, the degree of substitution is 3 (with substitution of all three OH groups of a glucose unit as the monomeric building block of the starch). The actual degree of substitution appears to be in the range of about 0.2 to 0.4; i.e., one OH group is substituted in approximately every fourth glucose unit (not taking crosslinking into account). This degree of substitution results from the values for the bound salt.

After Steps 1 and 2 have been performed, neutralization is carried out as Step 3, using an acid. Preferably, said acid is selected from the group consisting of succinic acid, phosphoric acid, hydrochloric acid, sulfuric acid, and citric acid. The selection of the acid has an effect on inorganic and organic substances that can be detected as residues in the modified starch. Most preferred according to the invention is phosphoric acid.

Accordingly, the pharmaceutical composition may further comprise an organic or inorganic salt resulting from the neutralization step, wherein said organic or inorganic salt is selected from the group consisting of succinate, phosphate, chloride, sulphate, and citrate.

### B) Carboxymethylated starch comprising citric acid and/or a salt of citric acid

In a further aspect, the invention provides a pharmaceutical composition comprising a carboxymethylated starch with the characteristics described in item A) above, and 1 to 20 percent by weight citric acid and/or a salt of citric acid relative to the weight of the carboxymethylated starch.

It has been found that a pharmaceutical composition a carboxymethylated starch and 1 to 20 percent by weight citric acid and/or a salt of citric acid relative to the weight of the carboxymethylated starch shows superior effects in reducing and/or inhibiting postoperative pain and in the treatment of disorders associated with the surgery of mesenchymal organs or tissues, of organs in serous cavities or of epithelial tissue.

The pharmaceutical composition of the invention comprises carboxymethylated starch in the form of powder particles, which preferably comprises a highly soluble citrate salt, wherein easy solubility is defined as a solubility of 100 g/l to 1000 g/l citrate in water at a temperature of 25 °C. It is crucial for the pharmaceutical effect that free citrate ions are present in the pharmaceutical composition after the addition of an, preferably aqueous, liquid.

By adding an aqueous liquid, the carboxymethylated starch of the pharmaceutical composition forms a gel, which also comprises citrate ions from citric acid or from a highly soluble citrate salt.

The citric acid and/or the citrate salt may be included in the pharmaceutical composition in an amount of 1% to 20% of the total weight of the carboxymethylated starch. The content of citric acid and/or a salt of citric acid relative to the weight of the carboxymethylated starch is preferably 1 to 10 percent by weight, more preferably 1 to 8 percent by weight, most preferably 2 to 5 percent by weight.

In a preferred embodiment, the pharmaceutical composition of the invention comprises a carboxymethylated starch and 1 to 20 percent by weight of a salt of citric acid relative to the weight of the carboxymethylated starch, wherein said salt of citric acid has a solubility in water of greater than 100 g/l, preferably greater than 200 g/l, more preferably greater than 300 g/l, most preferably greater than 400 g/l, at a temperature of 25°C.

Said salt of citric acid is preferably selected from the group consisting of a sodium citrate, a lithium citrate, a potassium citrate, a calcium citrate and a magnesium citrate. Most preferably, said salt of citric acid is trisodium citrate, which is biocompatible and which has a solubility of 425 g/l at a temperature of 25°C.

Further most preferably, said citrate from citric acid or a citrate salt, especially trisodium citrate is located on the surface of the particles of the carboxymethylated starch.

The high solubility of the citrate salt and the location on the surface of the starch particles enable a fast dissolution of the citrate salt and a fast delivery of the dissolved citrate ions for unfolding of the pharmacological effect, namely the inhibition of the formation of polyfibrin during blood coagulation.

The pharmaceutical composition is advantageously present in the form of a gel. In addition, the pharmaceutical composition according to the invention advantageously further comprises water and/or at least one salt, for example and in particular sodium chloride or potassium chloride.

In a preferred embodiment, the pharmaceutical composition of the invention comprises carboxymethylated starch and 1 to 20 percent by weight citric acid and/or a salt of citric acid relative to the weight of the carboxymethylated starch, and, optionally water.

In a further preferred embodiment, the pharmaceutical composition of the invention comprises carboxymethylated starch and 1 to 20 percent by weight citric acid and/or a salt of citric acid relative to the weight of the carboxymethylated starch, water and/or at least one salt, for example and in particular sodium chloride or potassium chloride.

### C) Pharmaceutical composition comprising a pain medication

In order to solve the problem of the invention, the invention provides a pharmaceutical composition comprising a carboxymethylated starch or epichlorohydrin-modified starch with the characteristics described in item A) or item B) above, and a pain medication.

Usually, the pharmaceutical composition of the invention comprises a pain medication in a dose range between 100 mg and 2,400 mg. Preferably, the pharmaceutical composition of the invention comprises a dose of the pain medication which is selected from 800 mg, 1,600 mg and 2,400 mg. More preferably, the pharmaceutical composition of the invention comprises a dose of the pain medication, which is selected from 100 mg, 200 mg, 300 mg, 400 mg, 500 mg, 600 mg, 800 mg, 1,000 mg, 1,200 mg, 1,400 mg, 1,600 mg, 1,800 mg, 2,000 mg, 2,200 mg and 2,400 mg.

Preferably, the pain medication comprised in the pharmaceutical composition of the invention is at least one local anesthetic.

Said at least one local anesthetic is preferably selected from the group consisting of lidocaine, cocaine, procaine, and, ambucaine, amethocaine, amylocaine, articaine, betoxycaine, benzocaine, bupivacaine, levo-bupivacaine, butacaine, butanilicicaine, butoxycaine, butyl aminobenzoate, carticaine, cinchocaine, clibucaine, clormecaine, chloroprocaine, cyclomethycaine, dibucaine, dimethocaine, levo-etidocaine, etidocaine, dextro-etidocaine, beta-eucaine, etidocaine, fomocaine, hexylcaine, hydroxyprocaine, hydroxytetracaine, leucinocaine, levo-mepivacaine, mepivacaine, meprylcaine, metabutoxycaine, myrtecaine, octacaine, orthocaine, oxethazaine, oxybuprocaine, parethoxycaine, phenacaine, piperocaine, piridocaine, prilocaine, procaine, propanocaine, proparacaine, propipocaine, proxymetacaine, propoxycaine, pseudococaine, pyrrocaine, risocaine, ropivacaine, levo-ropivacaine, tetracaine, tolycaine, trimecaine, and vadocaine.

In most preferred embodiment, said at least one local anesthetic comprised in the pharmaceutical composition of the invention is ropivacaine.

Accordingly, the invention provides a pharmaceutical composition which comprises a carboxymethylated starch or epichlorohydrin-modified starch with the characteristics described in item A) or item B) above, and a ropivacaine dose, wherein said ropivacaine dose is in the range of 100 to 2,400 mg, preferably selected from 100 mg, 200 mg, 300 mg, 400 mg, 500 mg, 600 mg, 800 mg, 1,000 mg, 1,200 mg, 1,400 mg, 1,600 mg, 1,800 mg, 2,000 mg, 2,200 mg and 2,400 mg. More preferred is a dose selected from 800 mg, 1,600 mg and 2,400 mg ropivcaine. Most preferred in the pharmaceutical composition of the invention is a ropivacaine dose in the range between 200 mg and 800 mg.

Ropivacaine is produced by several manufacturers, such as Fresenius-Kabi, B.Braun, Aspen, Noridem, etc. Usually, ropivacaine is distributed as solution in the following concentrations:
2 mg/ml (0.2%),
5 mg/ml (0.5%),
7.5 mg/ml (0.75%) and
10 mg/ml (1.0%).

The invention can make use of these commercially available ropivacaine solutions to prepare the pharmaceutical composition of the invention. Furthermore, the invention provides ropivacaine solutions in the following concentrations for the preparation of the pharmaceutical composition of the invention:
20 mg/ml (2.0 %),
30 mg/ml (3.0 %),
40 mg/ml (4.0 %),
50 mg/ml (5.0 %), and
60 mg/ml (6.0 %).

Ropivacaine has the formula 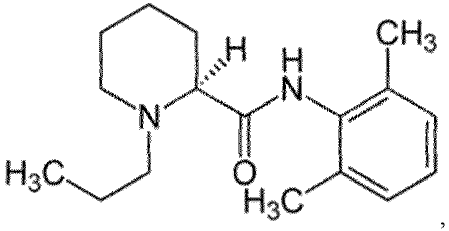 and the chemical name is (S)-1-Propyl-2',6'-dimethyl-2-piperidylcarboxyanilide. Ropivacaine is available as free base (CAS no. 84057-95-4), hydrochloride salt (CAS no. 98717-15-8) and hydrochloride monohydrate (CAS no. 132112-35-7).

Further ingredients of the ropivacaine solutions are sodium chloride, hydrochloric acid (e.g. 0.36%, for pH adjustment), sodium hydroxide (e.g. 0.4% for pH adjustment), and water.

Typically, the ropivacaine solutions comprise ropivacaine as ropivacaine hydrochloride or as ropivacaine hydrochloride monohydrate.

The pharmaceutical composition of the invention can easily be prepared by a simple process comprising adding carboxymethylated starch or epichlorohydrin-modified starch with the characteristics described in item A) or item B) above to a ropivacaine solution, mixing both components, and incubating the resulting suspension until a gel is formed. Incubation is preferably performed at room temperature. Gel formation starts immediately. The final gel is formed within 1 minute.

Typically, an amount in the range of 1 g to 10 g, preferably 5 g of a carboxymethylated starch or epichlorohydrin-modified starch with the characteristics described in item A) or item B) above is mixed with 20 to 100 ml ropivacaine solution, preferably 40 ml ropivacaine solution to prepare one dose of the pharmaceutical composition of the invention.

For preparing pharmaceutical compositions of the invention with the preferred ropivacaine dose strengths, the following amounts of the ingredients are mixed:

| **Ropivacaine dose (as hydrochloride or hydrochloride monohydrate)** | **Ropivacaine solution, 40 ml** | **Starch product according to item A) or B) described hereinabove** | **Resulting gel volume** |
|---|---|---|---|
| 200 mg | 5 mg/ml (0.5 %) | 5 g | 41.5 ml |
| 300 mg | 7.5 mg/ml (0.75 %) | 5 g | 41.5 ml |
| 400 mg | 10 mg/ml (1.0 %) | 5 g | 41.5 ml |
| 600 mg | 15 mg/ml (1.5 %) | 5 g | 41.5 ml |
| 800 mg | 20 mg/ml (2.0 %) | 5 g | 41.5 ml |
| 1,200 mg | 30 mg/ml (3.0 %) | 5 g | 41.5 ml |
| 1,600 mg | 40 mg/ml (4.0 %) | 5 g | 41.5 ml |
| 2,000 mg | 50 mg/ml (5.0 %), | 5 g | 41.5 ml |
| 2,400 mg | 60 mg/ml (6.0 %), | 5 g | 41.5 ml |

The Scientific Working Group on Regional Anesthesia of the German Society of Anesthesiology and Intensive Care Medicine recommends in the S1 guideline (AWMF register number 001-044, Prevention & therapy of systemic local anesthetic intoxication (LAST)) the following maximum ropivacaine doses:
Maximum single injection: 225 mg
Cumulative maximum dose: 800 mg/24 h

The maximum recommended doses as single doses for local application differ from country to country and range from 200 mg to 330 mg. There are, e.g. studies from the USA on joint replacements in which up to 1,000 mg ropivacaine is injected into the tissue at the end of the surgery (knee joint replacement).

CNS toxicity threshold of 0.60 µg/ml free plasma ropivacaine concentration has been described (minimum 0.34 mg µg/ml and maximum 0.85 µg/ml), with cardiovascular system being more resistant to the effects of local anaesthetics compared with CNS (Knudsen K, et al., Central nervous and cardiovascular effects of i.v. infusions of ropivacaine, bupivacaine and placebo in volunteers. Br J Anaesth 1997; 78: 507-14). However, such high plasma concentrations are reached only in case of i.v. infusions of ropivacaine. Following a local administration of ropivacaine, much lower plasma concentrations are reached, which are non-toxic. Local administrations of doses of 400 mg and 800 mg were found to be safe (Gromow, K. et al., Population pharmacokinetics of ropivacaine used for local infiltration anaesthesia during primary total unilateral and simultaneous bilateral knee arthroplasty. British Journal of Anaesthesia, 126 (4): 872e880 (2021)).

As it has been shown in practice during the treatment of hysterectomy and ablation as well as excision or resection of endometriosis that administration of the pharmaceutical composition of the invention comprising a dose of 200 mg ropivacaine achieves best results in pain treatment in patients who received a hysterectomy or an ablation, excision or resection of endometriomas.

For pain treatment after other surgeries, ropivacaine doses of up to 1.600 mg can be administered at once locally without creating toxic plasma levels because of the sustained release of ropivacaine from the pharmaceutical composition of the invention.

The pharmaceutical composition of the invention has an carrier effect and shows a sustained release of the pain medication. Preferably, the pain medication is released from the pharmaceutical composition over a period of 12 to 72 hours, preferably 12 to 48 hours, , most preferably 24 to 48 hours.

On the other hand, the pharmaceutical composition of the invention is of great advantage, because a single dose of 200 mg ropivacaine, which is below the recommendation of the guidelines for single injections (225 mg as maximum single dose) is sufficient for pain relief in patients after a surgery.

In a further embodiment, the pharmaceutical composition of the invention comprises at least one vasoconstrictor.

Vasoconstriction is the narrowing of the blood vessels resulting from contraction of the muscular wall of the vessels, in particular the large arteries and small arterioles. The process is the opposite of vasodilation, the widening of blood vessels. The process is particularly important in controlling hemorrhage and reducing acute blood loss. When blood vessels constrict, the flow of blood is restricted or decreased, thus retaining body heat or increasing vascular resistance. This makes the skin turn paler because less blood reaches the surface, reducing the radiation of heat. On a larger level, vasoconstriction is one mechanism by which the body regulates and maintains mean arterial pressure.

Medications causing vasoconstriction, also known as vasoconstrictors, are one type of medicine used to raise blood pressure. Generalized vasoconstriction usually results in an increase in systemic blood pressure, but it may also occur in specific tissues, causing a localized reduction in blood flow. The extent of vasoconstriction may be slight or severe depending on the substance or circumstance. Medications that cause vasoconstriction include antihistamines, decongestants, and stimulants.

Suitable vasoconstrictors for use in the pharmaceutical composition of the invention are selected from the group consisting of oxymetazoline, xylometazoline, phenylephrine, ephedrine, tetrahydrozoline, naphthazoline, and tramazoline including their pharmaceutically acceptable acid addition salts, such as their hydrochlorides or nitrates.

### Mode of administration to a subject

The pharmaceutical composition of the invention is administered as a gel directly after a surgery and directly on the wound area or site such that the wound or trauma area or site is covered by said pharmaceutical composition.

More specifically, the methods of the invention comprise the following steps:
i) providing a powder of starch particles of carboxymethylated starch or epichlorohydrin-modified starch with the characteristics as described in item A) or item B) above or a combination or mixture thereof;
ii) premixing the powder of Step i) with an aqueous liquid comprising a pain medication;
iii) forming a gel; and
iv) administering the gel of Step iii) on the wound or trauma area or site in a subject after surgery.

In an alternative embodiment, the method of the invention comprises the following steps:
i) providing a pharmaceutical composition which comprises a gel comprising carboxymethylated starch or epichlorohydrin-modified starch with the characteristics as described in item A) or item B) above or a combination or mixture thereof, and a pain medication; and
ii) administering the gel of Step i) on the wound or trauma area or site in a subject after surgery.

When the pharmaceutical composition used in the above described methods of the invention comprises a combination of carboxymethylated starch and epichlorohydrin-modified starch, the weight ratio of carboxymethylated starch to epichlorohydrin-modified starch is preferably in the range of 99 : 1, more preferably in the range of 90 : 10, 80 : 20, 70 : 30 or 60 : 40, most preferably 50 : 50.

In an alternative embodiment, when the pharmaceutical composition used in the above described methods of the invention comprises a combination of carboxymethylated starch and epichlorohydrin-modified starch, the weight ratio of epichlorohydrin-modified starch to carboxymethylated starch is preferably in the range of 99 : 1, more preferably in the range of 90 : 10, 80 : 20, 70 : 30 or 60 : 40, most preferably 50 : 50.

The aqueous liquid is preferably selected from the group consisting of water, and saline solution, such as isotonic saline solution, or hypotonic, isotonic, or hypertonic saline solution, as the liquid phase. The aqueous liquid can contain one or more cations selected from the group consisting of sodium, potassium, ammonium, magnesium, calcium, iron(II), iron(Ill), aluminum; and/or one or more anions selected from the group consisting of fluoride, chloride, bromide, iodide, oxide, sulfide, carbonate, sulfate, phosphate, nitrate, chromate, permanganate, hexacyanoferrate(II). Ringer's solution, Ringer's acetate solution, and Ringer's lactate solution may also be used as the liquid phase.

In a preferred embodiment, further ingredients of the aqueous solution comprising the pain medication are sodium chloride, hydrochloric acid (e.g. 0.36%, for pH adjustment), and sodium hydroxide (e.g. 0.4% for pH adjustment).

Accordingly, the pharmaceutical composition, when in gel form, may further include one or more cations selected from the group consisting of sodium, potassium, ammonium, magnesium, calcium, iron(II), iron(Ill), and aluminum; and/or further including one or more anions selected from the group consisting of fluoride, chloride, bromide, iodide, oxide, sulfide, carbonate, sulfate, phosphate, nitrate, chromate, permanganate, hexacyanoferrate(II), acetate and lactate.

Most preferably, the aqueous liquid is water or isotonic saline.

In the methods of the invention as described hereinbefore, the aqueous liquid, most preferably water or isotonic saline, is suitably added in a volume of 1-40 ml per gram powder of the pharmaceutical composition. An amount of aqueous liquid is added to the starch powder, sufficient to form a translucent gel.

The pharmaceutical composition of the invention is applicable in veterinary and human medicine. The subject is preferably a mammal. Most preferably, the subject is a human and the pharmaceutical composition of the invention is applied in human medicine.

### Mode of action of the pharmaceutical composition

It is presumed that the excellent properties are based on the following mechanisms:
The active form of ropivacaine is its free base. Ropivacaine free base is a strong base and is active in basic pH environments with best efficacy at a pH around 8.0. The water solubility of ropivacaine free base is weak. Accordingly, ropivacaine is provided as hydrochloride salt to increase its water solubility for pharmaceutical applications. The commercially available ropivacaine hydrochloride solutions have an acidic pH value in the range between 4.0 and 6.0. In this acidic pH range, ropivacaine shows a strongly reduced analgesic activity. After administration to a subject in need thereof, a natural equilibration occurs in the treated tissue until the physiological pH of about 7.4 is reached and ropivacaine can unfold its effect. Accordingly, there is a lag phase until the onset of the effect, when ropivacaine is administered as a hydrochloride solution. Beyond that, the duration of this lag phase is patient dependent.

The carboxymethylated starch of the invention has a pH value in the range between 5.5 and 7.5 in solution, with a mean value at pH 6.5. This pH value is closer at the optimum pH value of ropivacaine than that of the ropivacaine hydrochloride solution. Accordingly, when the ropivacaine hydrochloride solution is mixed together with the carboxymethylated starch in the pharmaceutical composition of the invention, the lag phase to reach optimum analgesic efficacy of ropivacaine can be drastically reduced.

### Advantages associated with the use of local anesthetics and carboxymethylated starch in the present invention

Local anesthetics, such as ropivacaine, have an intensive effect on afferent nerve fibers and exert their effect primarily directly in the area of the body affected by pain. The lower pain intensity facilitates physiotherapy, coughing, breathing, leads to a lower incidence of pneumonia, atelectasis or respiratory insufficiency, less nocturnal hypoxemia, less postoperative fatigue, less nausea, cognitive impairment and sedation. In the meantime, a large number of randomized, controlled studies and several meta-analyses have demonstrated the advantages of analgesia with local anaesthetics compared to systemic analgesia or analgesia with opioids.

The general side effects of local anesthetics are significantly lower than those of basic analgesics. However, there is a certain cardiotoxicity (prolongation of AV conduction with arrhythmia), which is essentially only problematic if systemic intravenous administration has been applied accidentally.

Local anesthetics have a duration of action of a few hours. They are rapidly absorbed after local infiltration into the tissue. If painkillers are required for longer periods, infusion systems must be used, which is associated with equipment and personnel costs and the risk of ascending infection along the infusion tubes.

In the present invention, starch particles (carboxymethylated starch) and solutions containing local anesthetics as active ingredients are mixed. Particles and active ingredient solution form a gel (hereinafter: pain gel).

The pain gel is applied to the affected areas of pain, allowing the active ingredient to develop its effect locally. The carboxymethylated starch of the pain gel is broken down over > 2 days, such as over 4 to 5 days, so that the active substance contained in the gel is present and available in the area of pain for > 2 days, e.g. for 4 to 5 days.

When the pain gel is applied to mesothelium or mesothelial-like tissue (peritoneum, pericardium, pleura, synovial membranes, tendon sheath membranes), the starch particles loaded with active ingredient penetrate under the epithelium into the underlying stratum proprium (connective tissue layer), where the afferent nerve fibers of the pain sensation are located. The degradation of the starch takes place over > 2 days, and the active ingredient, the local anesthetic, becomes available in the same way.

The use of the pharmaceutical composition of the invention has the following further advantages:
Advantage 1: Systemic intravenous injection of the pain gel is excluded.

As the active ingredient is applied as a gel to the areas affected by pain, systemic intravascular application of the local anaesthetic is excluded and the risk of a cardiotoxic effect is minimized.

Advantage 2: No loss of active ingredient due to large-scale diffusion.

Due to its adsorption to the starch particles, the active ingredient does not diffuse over a large area into the surrounding tissue and is not lost through diffusion in areas where there is no need for pain relief. More painkiller is available at the site where it is needed.

Advantage 3: Reduced peak plasma concentrations through prolonged release of the active ingredient due to its adsorption on the starch particles.

The local anesthetic is adsorbed to the starch particles within the pain gel and is not available for rapid systemic absorption within hours compared to tissue infiltration or direct administration of the active ingredient solution to mesothelial surfaces. This reduces the problem of peak formation of the active ingredient in plasma.

Advantage 4: Larger quantities of active substance can be applied initially as a depot.

The active substance adsorbed in the pain gel remains in the gel as a depot. Larger quantities of active ingredient, e.g. one, two or more times the cumulative daily dose, can be administered initially.

Advantage 5: Prolonged availability of the active substance in the pain areas over a longer period (> 2 days, e.g. 4 to 5 days).

The availability of the active ingredient is analogous to the degradation of the starch and is available for the duration of the degradation period.Pharmaceutical uses

The invention further relates to the use of the pharmaceutical composition in the treatment pain after surgery.

Accordingly, the invention provides the pharmaceutical composition of the invention for use in the treatment of pain after a surgery.

Moreover, the invention provides the use of a pharmaceutical composition of the invention for the preparation of a medicament for the treatment of pain after a surgery.

In a further embodiment, the invention relates to a method of treating pain after a surgery, said method comprising administering to a subject in need thereof a pharmaceutical composition of the invention.

In these methods or uses, the pharmaceutical composition of the invention is administered locally as a gel on a wound site or area following a surgery.

The pharmaceutical composition, use or method according to the invention is suitable for treating pain after a surgery, wherein said surgery is selected from cardio-thoracic surgeries, ear-nose-throat surgeries, general surgeries, gynecological surgeries, neurosurgeries, surgeries in orthopedics and traumatology, urological surgeries, and vascular surgeries.

Examples of cardio-thoracic surgeries are open lung resection (atypical, segmental, lobar) and thoracoscopic lung resection (atypical, segmental, lobar).

Examples of ear-nose-throat surgeries are paranasal sinus surgery (several sinuses), parotidectomy, rhinoplasty and submandibular gland surgery.

Examples of general surgeries are adrenal gland surgery (lap), adrenal gland surgery (open), anal surgery, fistula, anal surgery, hemorrhoids (plastic reconstruction), anal surgery, hemorrhoids (stapler procedure), anal surgery, hemorrhoids, excision (e.g. milligan-morgan), appendectomy (lap), appendectomy (open), cholecystectomy (lap), cholecystectomy (open), colorectal surgery, left hemicolectomy (open), colorectal surgery, proctocolectomy (open), colorectal surgery, resection of rectum with sphincter preservation (lap), colorectal surgery, resection of rectum with sphincter preservation (open), colorectal surgery, resection of rectum without sphincter preservation, colorectal surgery, right hemicolectomy (lap), colorectal surgery, right hemicolectomy (open), colorectal surgery, sigmoidectomy (lap), colorectal surgery, sigmoidectomy (open), small bowel resection (open), enterostomy as independent procedure, excision of solitary lymph nodes (axillary), excision of solitary lymph nodes (cervical), excision of solitary lymph nodes, inguinal (open), extended excision of cutaneous and subcutaneous tissue, gastric surgery, epigastric hernia repair (lap), gastric surgery, epigastric hernia repair (open), gastric surgery, fundoplication (lap), gastric surgery, gastrectomy (total or subtotal), gastric surgery, vertical sleeve gastrectomy with duodenal switch (lap), gastric surgery, vertical sleeve gastrectomy with duodenal switch (open), gastric surgery, bariatric surgery, implantation or exchange of an adjustable gastric band (lap), hernia, incisional hernia repair, with alloplastic material (lap), hernia, incisional hernia repair, with alloplastic material (open), hernia, incisional hernia repair, with reconstruction, hernia, incisional hernia repair, without reconstruction, hernia, inguinal hernia - femoral hernia repair (open), hernia, inguinal hernia repair (w or w/o mesh) (endoscopic), hernia, inguinal hernia repair (w or w/o mesh) (lap), hernia, inguinal hernia repair (w or w/o mesh) (open), hernia, umbilical hernia repair (lap), hernia, umbilical hernia repair (open), kidney transplantation, liver resection (anatomical) (seg., biseg., hemihepatectomy), liver resection (atypical), w or w/o cholecystectomy (lap), liver resection (atypical), w or w/o cholecystectomy (open), splenectomy (open), pancreatectomy (partial - w or w/o resection of duodenum), pancreatectomy (partial - whipple), thyroid (total, hemi, partial resection), and pilonidal sinus surgery.

Examples of gynecological surgeries are breast augmentation, breast conservation surgery (local, ductal, lump resections), breast conservation surgery (segment; quadrant resection), breast reconstruction (muscle or skin flap), breast, mastectomy (w or w/o axillary lymphadenectomy), breast, reduction mammoplasty, caesarean section, extrauterine pregnancy (lap), fallopian tube surgery (insufflation, chromopertubation) (lap), hysterectomy (open, vaginal), hysterectomy (subtotal) (lap), hysterectomy (subtotal) (open), myomectomy (lap, vaginal laparoscopically assisted), myomectomy (open), oophorectomy and salpingo-oophorectomy (lap), oophorectomy and salpingo-oophorectomy (open), transvaginal sling suspension of bladder neck, vulva surgery (incision, bartholin's gland, local excision), removal of ovarian cysts (lap), caesarean section, and female pelvic floor reconstruction.

Examples of neurosurgeries are, laminectomy, hemilaminectomy (cervical, ventral, 1-2 seg.), laminectomy, hemilaminectomy (lumbar, 1-2 segments), spinal canal decompression (1 segment), spinal canal decompression (2 segments), skull and/or brain surgery.

Examples of surgeries in orthopedics and traumatology are, amputation, lower leg, amputation, metatarsophalangeal, amputation, toe, amputation, trans-fermoral, amputation, transmetatarsal, arthrodesis (ankle joint), arthrodesis (foot joint), arthrodesis (metacarpophalangeal, interphalangeal joints), arthrodesis (toe joint), arthroscopic revision (wrist joint), arthroscopic surgery (ankle joint), arthroscopic surgery (hip joint), arthroscopic surgery (knee ligaments), arthroscopic surgery (knee) (excl. ligaments and meniscus), arthroscopic surgery (shoulder joint), arthroscopic surgery (shoulder ligaments), closed reduction with internal fixation (clavicle), closed reduction with internal fixation (fibula), closed reduction with internal fixation (humerus), closed reduction with internal fixation (metacarpal bones), closed reduction with internal fixation (radius), closed reduction with internal fixation (tibia), closed reduction, internal fixation (femur or neck of femur), hand resection arthroplasty, hand tendon repair, open reconstruction (ankle joint ligaments), open reconstruction (shoulder joint ligaments), open reconstruction, refixation (knee ligaments), open reduction (acetabulum and head of femur), open reduction (calcaneus), open reduction (carpal bones), open reduction (clavicle), open reduction (distal femur), open reduction (distal fibula), open reduction (distal humerus), open reduction (distal radius), open reduction (distal tibia), open reduction (femur shaft), open reduction (fibula shaft), open reduction (finger), open reduction (humerus shaft), open reduction (metacarpal bone), open reduction (metatarsal bone), open reduction (patella), open reduction (pelvic rim and ring), open reduction (proximal femur), open reduction (proximal humerus), open reduction (proximal radius), open reduction (proximal tibia), open reduction (proximal ulna), open reduction (radius shaft), open reduction (tibia shaft), open reduction (ulnar shaft), removal of osteosynthetic material (clavicle), removal of osteosynthetic material (femur w/o femoral neck), removal of osteosynthetic material (fibula), removal of osteosynthetic material (radius), removal of osteosynthetic material (tibia w or w/o fibula), removal of osteosynthetic material (ulnar), replacement, hip joint, replacement, hip joint (revision, exchange, removal), replacement, knee joint, replacement, knee joint (revision, exchange, removal), replacement, shoulder joint, replacement, shoulder joint, partial (humerus), spinal fusion, dorsal (1-2 segments), spinal fusion, dorsal (3 or more segments), spinal fusion, ventral (1-2 segments), and spinal reconstruction (complex).

Examples of urological surgeries are, cystectomy (total) (urinary bladder), nephrectomy (lap), nephrectomy (open), orchidectomy, radical prostatectomy (lap), radical prostatectomy (open), and testicular hydrocele surgery.

Examples of vascular surgeries are creation of av-fistula (e.g. cimino-shunt), endarterectomy (carotid and femoral arteries), shunt or bypass (femoral and/or popliteal artery), and varicose veins surgery (ligation, excision, stripping).

### Example 1: Starch products used in pharmaceutical compositions of the invention

The following defined starch products were used to prepare example compositions of the invention:
**A) Carboxymethylated starch which had the following characteristics:**

| | |
|---|---|
| Chemical name: | Starch, carboxymethyl ether, sodium salt, present as sodium glycolate |
| Appearance: | white or almost white fine, free-flowing powder |
| Odour: | neutral |
| Molecular weight: | in the range of 500,000 daltons to 11,000,000 daltons |
| Particle size: | in the range of 30 µm to 100 µm |
| Water content: | 3.9 % (w/w) |
| Sodium chloride: | 4.0 (w/w) |
| Sodium glycolate: | < 2.0 (w/w) |
| Degree of crosslinking: | 34 % |
| Crosslinking agent: | Na₃P₃O₉ (sodium trimetaphosphate) |

**B) Carboxymethylated starch with citric acid or a salt of citric acid which had the following characteristics:**

| | |
|---|---|
| Chemical name: | Starch, carboxymethyl ether, sodium salt, present as sodium glycolate |
| Appearance: | white or almost white fine, free-flowing powder |
| Odour: | neutral |
| Molecular weight: | in the range of 500,000 daltons to 11,000,000 daltons |
| Particle size: | in the range of 30 µm to 100 µm |
| Water content: | 3.9 % (w/w) |
| Sodium chloride: | 4.0 (w/w) |
| Sodium glycolate: | < 2.0 (w/w) |
| Sodium citrate: | 3.2 % (w/w) |
| Degree of crosslinking: | 34 % |
| Crosslinking agent: | Na₃P₃O₉ (sodium trimetaphosphate) |

Comparative mass spectrometry and IR spectroscopy measurements of starch products A) and b) were performed. IR spectroscopy results show that both samples are constituent with carboxymethyl starch sodium salt. The IR spectroscopy analysis did not find any differences between the two samples except for the five signals for trisodium citrate in the sample of starch product B). Results of the mass spectrometry measurements further confirm that the starch product B) contains sodium citrate while starch product A) does not. Mass spectrometry did not reveal any further differences as well.

### Example 2: Examples of pharmaceutical compositions of the invention

The following pharmaceutical compositions were prepared comprising a carboxymethylated starch of item A) or item B) as described in Example 1:

| **Ropivacaine dose (as hydrochloride or hydrochloride monohydrate)** | **Ropivacaine solution, 40 ml (B.Braun)** | **Starch product according to item A) or B) described in Example 1** | **Resulting gel volume** |
|---|---|---|---|
| 200 mg | 5 mg/ml (0.5 %) | 5 g A) | 41.5 ml |
| 300 mg | 7.5 mg/ml (0.75 %) | 5 g A) | 41.5 ml |
| 400 mg | 10 mg/ml (1.0 %) | 5 g A) | 41.5 ml |
| 600 mg | 15 mg/ml (1.5 %) | 5 g A) | 41.5 ml |
| 800 mg | 20 mg/ml (2.0 %) | 5 g A) | 41.5 ml |
| 200 mg | 5 mg/ml (0.5 %) | 5 g B) | 41.5 ml |
| 300 mg | 7.5 mg/ml (0.75 %) | 5 g B) | 41.5 ml |
| 400 mg | 10 mg/ml (1.0 %) | 5 g B) | 41.5 ml |
| 600 mg | 15 mg/ml (1.5 %) | 5 g B) | 41.5 ml |
| 800 mg | 20 mg/ml (2.0 %) | 5 g B) | 41.5 ml |
| 1,200 mg | 30 mg/ml (3.0 %) | 5 g B) | 41.5 ml |
| 1,600 mg | 40 mg/ml (4.0 %) | 5 g B) | 41.5 ml |
| 2,000 mg | 50 mg/ml (5.0 %) | 5 g B) | 41.5 ml |
| 2,400 mg | 60 mg/ml (6.0 %) | 5 g B) | 41.5 ml |

The preparation of the example pharmaceutical compositions was performed by mixing 40 ml of ropivacaine solution of the desired strength and 5 g of starch product A) or B) to obtain 41.5 ml of gel volume. In this constellation, 1 g of starch absorbed a total of 8 ml of ropivacaine solution and the respective amount of ropivacaine.

## Claims

1. A pharmaceutical composition comprising
- a carboxymethylated starch or epichlorohydrin-modified starch,
- water,
- at least one pain medication selected from the group consisting of morphine, nonsteroidal anti-inflammatory drugs (NSAIDS), opioid analgesics, opioid/nonopioid combination analgesics, acetaminophen, local anesthetics, alpha-2 agonists, VR1 antagonists, and combinations thereof, and
- optionally a vasoconstrictor,
wherein said pharmaceutical composition is a solution, suspension or a gel.

2. The pharmaceutical composition of claim 1, further comprising 1 to 20 percent by weight of a compound selected from the group consisting of citric acid, a salt of citric acid, and a mixture thereof, relative to the weight of the carboxymethylated starch.

3. The method of claim 1 or 2, wherein said carboxymethylated starch is present as a sodium salt of a carboxymethyl ether of the starch.

4. The pharmaceutical composition according to any one of the preceding claims, wherein said carboxymethylated starch has a molecular weight in the range of 500,000 daltons to 11,000,000 daltons.

5. The pharmaceutical composition according to any one of the preceding claims, wherein the starch particles of said carboxymethylated starch have a particle diameter in the range of 30 µm to 100 µm.

6. The pharmaceutical composition according to any one of claims 2 to 5, wherein said salt of citric acid is on the surface of carboxymethylated starch particles.

7. The pharmaceutical composition according to any one of the preceding claims, wherein particles of said carboxymethylated starch are made up of about 4.5×10¹⁰ to 2.3×10¹² amylose molecules and 5.6×10⁷ to 1.3×10¹⁰ amylopectin molecules.

8. The pharmaceutical composition according to any one of the preceding claims, wherein carboxymethylated starch is crosslinked having a degree of crosslinking in the range of 25%

9. The pharmaceutical composition according any one of the preceding claims, wherein said pharmaceutical composition comprises said pain medication in the range of 100 mg to 2,400 mg.

10. The pharmaceutical composition according to any one of the preceding claims, wherein said pain medication comprised in said pharmaceutical composition is at least one local anesthetic selected from the group consisting of lidocaine, cocaine, procaine, and, ambucaine, amethocaine, amylocaine, articaine, betoxycaine, benzocaine, bupivacaine, levo-bupivacaine, butacaine, butanilicicaine, butoxycaine, butyl aminobenzoate, carticaine, cinchocaine, clibucaine, clormecaine, chloroprocaine, cyclomethycaine, dibucaine, dimethocaine, levo-etidocaine, etidocaine, dextro-etidocaine, beta-eucaine, etidocaine, fomocaine, hexylcaine, hydroxyprocaine, hydroxytetracaine, leucinocaine, levo-mepivacaine, mepivacaine, meprylcaine, metabutoxycaine, myrtecaine, octacaine, orthocaine, oxethazaine, oxybuprocaine, parethoxycaine, phenacaine, piperocaine, piridocaine, prilocaine, procaine, propanocaine, proparacaine, propipocaine, proxymetacaine, propoxycaine, pseudococaine, pyrrocaine, risocaine, ropivacaine, levo-ropivacaine, tetracaine, tolycaine, trimecaine, and vadocaine.

11. The pharmaceutical composition of claim 10, wherein said at last one local anesthetic is ropivacaine.

12. The pharmaceutical composition of claim 10 or 11, wherein said pharmaceutical composition is a gel, which comprises 5 g of a carboxymethylated starch or epichlorohydrin-modified starch, and which has a volume of 41.5 ml.

13. A process of preparing a pharmaceutical composition according to any one of the preceding claims, said process comprising the steps of
i) providing a powder of starch particles of carboxymethylated starch or epichlorohydrin-modified starch with the characteristics as claimed in any one of claims 1 to 8;
ii) premixing the powder of Step i) with an aqueous liquid comprising a pain medication as claimed in any one of claims 1, 10 and 11; and
iii) forming a gel.

14. The pharmaceutical composition according to any one of claims 1 to 12 for use in the treatment of pain after a surgery.

15. The pharmaceutical composition for use according to claim 14, wherein said surgery is selected from cardio-thoracic surgeries, ear-nose-throat surgeries, general surgeries, gynecological surgeries, neurosurgeries, surgeries in orthopedics and traumatology, urological surgeries, and vascular surgeries.
